(19) Europäisches Patentamt / European Patent Office / Office européen des brevets

(11) **EP 4 628 112 A1**

(12) **EUROPEAN PATENT APPLICATION**
published in accordance with Art. 153(4) EPC

(43) Date of publication:
**08.10.2025 Bulletin 2025/41**

(21) Application number: **23898252.4**

(22) Date of filing: **28.11.2023**

(51) International Patent Classification (IPC):
***A61L 26/00*** *(2006.01)*

(52) Cooperative Patent Classification (CPC):
**A61L 26/00**

(86) International application number:
**PCT/KR2023/019323**

(87) International publication number:
**WO 2024/117726 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.11.2022 KR 20220163650**

(71) Applicant: **Youth Bio Global Co., Ltd.**
**Seoul 08381 (KR)**

(72) Inventor: **YOO, Seung Ho**
**Seoul 05607 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **WOUND DRESSING MATERIAL COMPOSITION CONTAINING FUCOIDAN AND OLIVE TREE LEAF EXTRACT**

(57) The present invention relates to a wound dressing material composition capable of achieving a skin wound healing effect by facilitating the growth of stem cells in the epidermal stratum basale of the skin. The present invention provides「a wound dressing material composition comprising: 60-70 wt% of glycerin; 28-39 wt% of honey; 0.2-3.0 wt% of fucoidan; 0.2-3.0 wt% of an olive tree leaf extract; and 0.1-0.3 wt% of xanthan gum」.

FIG. 6

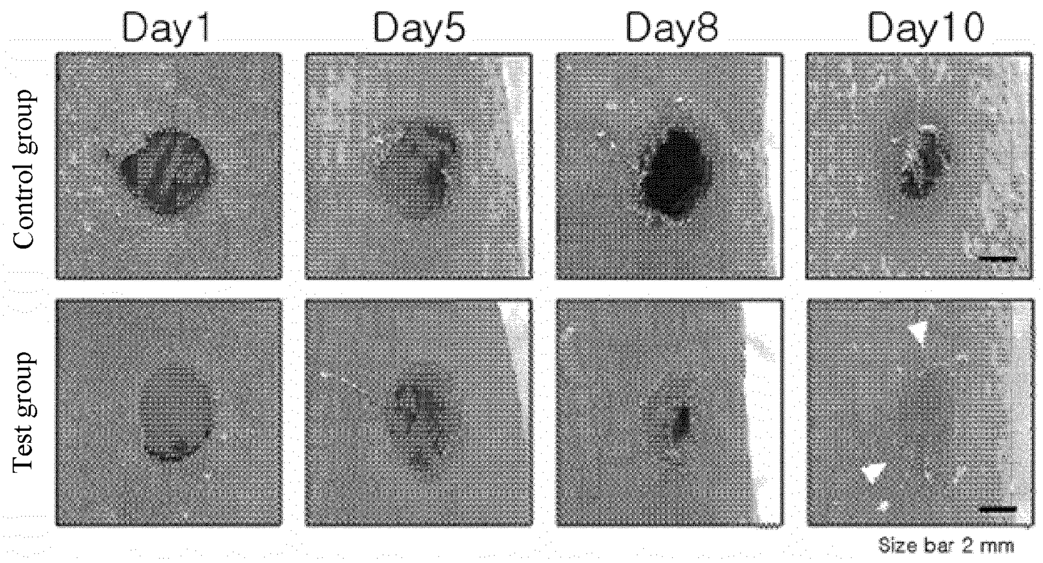


EP 4 628 112 A1

## Description

[TECHNICAL FIELD]

The present disclosure relates to a wound dressing composition that may achieve a skin wound healing effect by promoting the growth of epidermal basal layer stem cells of skin.

[BACKGROUND ART]

Korean Patent No. 10-2088767 is an invention regarding a "composition for increasing stem cell biological activity by using mixture 4F", and provides a composition for inhibiting aging, promoting proliferation, or inducing differentiation of stem cells containing fucoidan, tauroursodeoxycholic acid, oleuropein, and vascular endothelial growth factor as effective ingredients.

The "stem cells using mixture 4F" presented in the above patent not only acquire undifferentiated characteristics (stemness), but also have the effect of improving cell proliferation and migration ability, so that after stem cells are transplanted into the body, they may improve cell survival and engraftment rates and enhance vascular and tissue regeneration abilities, and thus may be utilized in various fields such as stem cell differentiation and prevention or treatment of ischemic diseases.

Meanwhile, human skin tissues are made up of the epidermis, dermis, and hypodermis. The epidermis may be divided into the stratum corneum, which is made up of dead cells, and the living epidermis, and the living epidermis may be divided into four layers (stratified epithelial tissues) of the stratum lucidum, the stratum granulosum, the stratum spinosum, and the basal layer (stratum basale).

When a wound occurs on the skin, the wounded area has to receive stem cells from the basal layer to heal the wound through stem cell division and growth. Accordingly, a wound dressing composition that may achieve a skin wound healing effect by promoting the growth of basal layer stem cells by utilizing Korean Patent No. 10-2088767 and related research results was examined.

[DETAILED DESCRIPTION OF THE INVENTION]

[TECHNICAL PROBLEM]

An objective of the present disclosure is to provide a wound dressing composition that may achieve a skin wound healing effect by promoting the growth of epidermal basal layer stem cells by using fucoidan and olive leaf extract.

[TECHNICAL SOLUTION]

The present disclosure provides a wound dressing composition including 60 to 70 wt% of glycerin, 28 to 39 wt% of honey, 0.2 to 3.0 wt% of fucoidan, 0.2 to 3.0 wt% of olive leaf extract, and 0.1 to 0.3 wt% of xanthan gum.

The fucoidan may be obtained by mixing dried seaweed stem powder or extract with purified water, followed by centrifugation, filtration, and collection, and the olive leaf extract may be obtained by subjecting a filtrate of crushed olive leaf powder or an extract from olive leaves to sterilization by heating at 120 to 125°C for 15 to 18 minutes.

A viscosity of the wound dressing composition may be adjusted to a range of 500 to 10,000 mPa·s depending on a content of xanthan gum.

[ADVANTAGEOUS EFFECTS OF THE INVENTION]

The present disclosure provides
a wound healing effect through the organic action of each component of the wound dressing composition and their optimal ratio, and specifically, the composition offers: 1) exudate drainage through the osmotic pressure of glycerin, 2) moisturizing effects from honey and antibacterial/antimicrobial effects due to enzyme-based spoilage prevention, 3) promotion of basal layer stem cell growth by fucoidan, and 4) due to organic operations of antioxidant, anti-inflammatory, antimicrobial, and inflammation-relieving effects from olive leaf extract, a skin wound heating effect may be obtained, and an appropriate viscosity is achieved, allowing a film to be maintained over the wound site by xanthan gum.

[DESCRIPTION OF THE DRAWINGS]

FIG. 1 is a graph depicting results of measuring the cytotoxicity of fucoidan and olive leaf extract (oleuropein) at different concentrations on adult HDFa cells and epidermal keratinocytes.

FIG. 2 is a SEM photograph and a graph depicting results of measuring the wound healing efficacy of fucoidan and olive leaf extract (oleuropein) at different concentrations on adult HDFa cells.

FIG. 3 is a SEM photograph and graph depicting results of measuring the wound healing efficacy of fucoidan and olive leaf extract (oleuropein) at different concentrations on human epidermal keratinocytes.

FIG. 4 is a graph depicting the wound recovery rate by date in a control group and an experimental group.

FIG. 5 is a graph depicting the recovery rate by date of an experimental group compared to a control group.

FIG. 6 is a photograph depicting the wound healing status by date in a control group and an experimental group.

[BEST MODE]

A wound dressing composition includes 60 to 70 wt% of glycerin; 28 to 39 wt% of honey; 0.2 to 3.0 wt% of fucoidan; 0.2 to 3.0 wt% of olive leaf extract; and 0.1 to 0.3 wt% of xanthan gum.

The fucoidan is obtained by mixing dried seaweed stem powder or extract with purified water, followed by centrifugation, filtration, and collection.

The olive leaf extract is obtained by subjecting a filtrate of crushed olive leaf powder or an extract from olive leaves to sterilization by heating at 120 to 125°C for 15 to 18 minutes.

The present disclosure relates to a wound dressing composition that utilizes substances known to promote skin regeneration when the skin barrier has been damaged due to wounds, burns, ulcers, contusions, post-surgical wounds, childbirth, chronic wounds, dermatitis, and the like, and aims to prevent infection and provide antioxidant effects by using relatively easily obtainable substances, of which safety has already been established.

Stem cells are cells that have the ability to self-replicate and differentiate into two or more cells, and the homeostasis of stratified epithelial tissues is maintained by the proliferative potential of the basal layer stem cells. Accordingly, when a wound occurs on the skin, the wounded area has to receive stem cells from the basal layer of the epidermal-dermal boundary area in order for the wound to be healed through the division and growth of stem cells.

The present disclosure applies fucoidan and olive leaf extract, which the culture medium compositions for a stem cell therapeutic agent studied through Korean Patent No. 10-2088767, to a wound dressing composition.

However, when a wound occurs on the skin, the supply and proliferation of stem cells from the basal layer may be hindered by exudates, microorganisms, and other contaminants that are present at the wound area, and thus, it is important that the wound dressing cleans the wound area, alleviates the heat and pain caused by the wound, and maintains a certain level of viscosity so that it does not easily run off when applied to the wound area.

The present disclosure has been derived in consideration of the above factors, and provides a wound dressing composition including: 60 to 70 wt% of glycerin; 28 to 39 wt% of honey; 0.2 to 3.0 wt% of fucoidan; 0.2 to 3.0 wt% of olive leaf extract; and 0.1 to 0.3 wt% of xanthan gum.

The wound dressing composition provided by the present disclosure includes glycerin and honey as the base materials, fucoidan and olive leaf extract as active ingredients for therapeutic effects, and xanthan gum as a thickening agent.

Glycerin ($C_3H_8O_3$) is a non-irritating substance, and is widely used as a main ingredient in enemas, lubricants, creams, eye drops, injectable solutions, etc., and is also widely utilized as a component in wound dressings.

Glycerin has high osmotic pressure, and thus, it contracts the cells in the body, allowing exudate to be discharged from the inside of the wound to the outside. In this case, microorganisms and other contaminants in the wound area are separated and discharged together, and the discharged exudate, microorganisms, and other contaminants are also blocked by glycerin from entering the body again. As a result, the inside of the wound is cleaned, promoting wound healing.

Furthermore, glycerin has a high binding force, so that when applied to a wound, it does not penetrate into the body tissues but remains outside the wound. When the glycerin is mixed with xanthan gum, which will be described later, a film is formed over the wound while its viscosity increases.

The wound dressing composition of the present disclosure contains 60 to 70 wt % of glycerin.

Honey is a liquid that bees eat and spit out from the nectar glands of flowers, and is a viscous liquid that is obtained by decomposing sucrose into fructose and glucose by bee enzymes. Honey does not spoil due to the osmosis phenomenon caused by its high sugar content and the anti-rot enzymes contained in flower nectar. Accordingly, when honey is applied to a wounded area of the skin, the moisture of bacteria moves to the honey due to the action of the anti-rot enzymes mentioned above and the osmosis phenomenon, so that it performs an anti-bacterial and anti-fungal function by drying and killing bacteria that have lost moisture.

Furthermore, honey forms a skin protecting layer to perform a skin soothing function of relieving the heat and pain of skin irritated by wounds, and a moisturizing function of retaining moisture in the skin to reduce discomfort such as heat, itchiness, and dryness, and to make the skin smooth.

The wound dressing composition of the present disclosure contains 28 to 39 wt % of honey.

The above fucoidan is a substance that provides flexibility to brown algae, allowing the algae to be protected from strong tidal currents. Fucoidan extracted from brown algae is a polysaccharide composed of a basic sugar called fucose and a sulfate group, and is known to have anticoagulant, antitumor, anti-ulcer, antibacterial, anti-inflammatory, antihypertensive, hepatocyte growth factor (HGF) production induction, antihyperglycemic, immune cell regulation, anti-allergic, and antiviral effects.

Because fucoidan contains a large amount of highly hydrophilic sulfate groups, it has high skin regeneration power, assists and activates the proliferative potential of the basal layer stem cells, and contributes to the maintenance of homeostasis of stratified epithelial tissues. That is, it promotes wound healing by helping the division and growth of basal layer stem cells in the epidermal-dermal boundary area.

One having a structure as illustrated in [Chemical Structure 1] below may be applied as the fucoidan.

[Chemical structure 1]

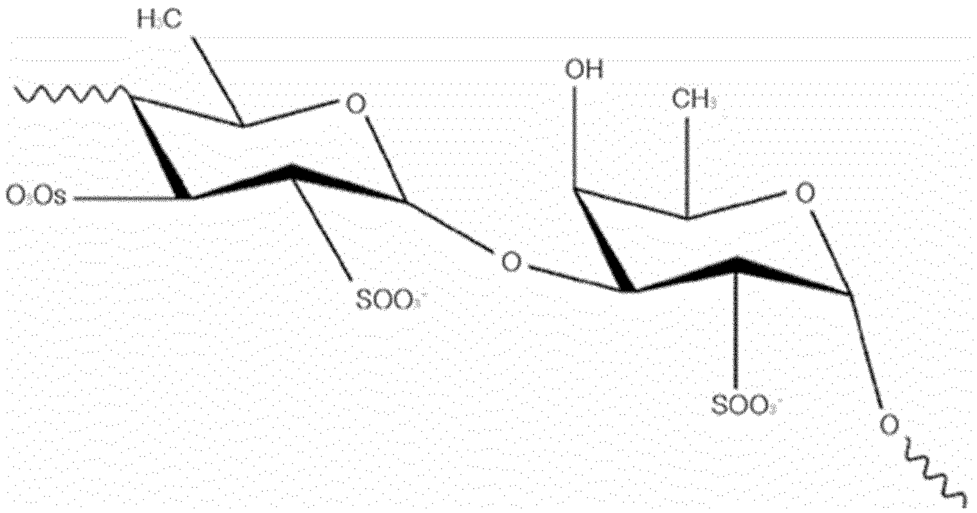

In the present disclosure, fucoidan was applied by mixing dried seaweed powder or extract with purified water, performing centrifugation three times, and then filtering it to recover it, and its molecular weight is formed in the range of 1,000 to 2,000,000 Da. The wound dressing composition of the present disclosure contains 0.2 to 3.0 wt % of the fucoidan.

Olive leaf extract is a component of oleuropein extracted from olive leaves, and has excellent antioxidant, anti-inflammatory, and antibacterial effects and also is effective in relieving inflammation, so that it is widely used as a skin protectant and natural antibiotic. The polyphenol that is present in olive fruits and olive leaves is collectively called oleuropein, and olive leaf extract contains more oleuropein than olive oil. One having a structure as illustrated in the following [Chemical Structure 2] may be applied as the olive leaf extract or oleuropein.

[Chemical structure 2]

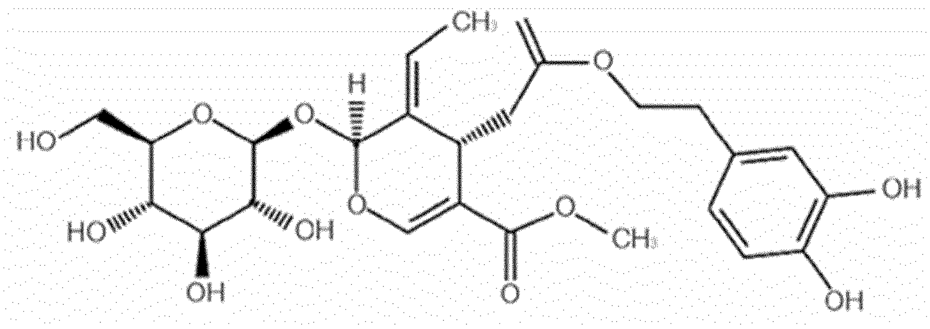

In the present disclosure, the wound healing effect was maximized through sterilization treatment by heating the filtered liquid of crushed olive leaf powder or the liquid extracted from olive leaf at 120 to 125 °C for 15 to 18 minutes.

The wound dressing composition of the present disclosure contains 0.2 to 3.0 wt % of the olive leaf extract.

Xanthan gum is obtained by purifying, drying, and crushing a highmolecular-weight polysaccharide gum

substance obtained by the pure culture fermentation of carbohydrates by using xanthomonas campestris in isopropyl alcohol, and is a mixture composed of sodium, potassium, and calcium salts of glucose, mannose, and glucuronic acid. Xanthan gum is an extracellular polysaccharide, of which a main backbone consists of β-1,4-linked glucose (cellulose), and every two glucose residues in the backbone are substituted with a trisaccharide side chain composed of mannose-glucuronic acidmannose. Mannose, which is bound to glucose, is acetylated. The xanthan gum is used as a food additive in foods as a stabilizer, thickener, binder, emulsifier, solidifier, foaming agent, etc.

The wound dressing composition of the present disclosure has a difference in viscosity depending on the concentration and content of glycerin and the content of honey, but by controlling the amount of xanthan gum added in the range of 0.1 to 0.3 wt %, the viscosity of the wound dressing composition may be controlled in the range of 500 to 10,000 mPa·s.

The above-mentioned wound dressing composition may be stirred at a speed of 80 to 300 rpm for 24 to 120 hours, and ensuring a homogenized mixture, it may be used as a medicine or a cosmetic composition, as well as a pharmaceutical product. Depending on the intended use, the wound dressing composition may be controlled to a pH range of 5 to 9, and may be manufactured in a translucent or opaque state.

The wound dressing composition may be commercialized by injecting 0.1 to 500 cc into a container, and the wound dressing composition may be sterilized by irradiating it with gamma rays while it is injected into the container.

Hereinafter, the results of our own tests using adult HDFa cells and keratinocytes will be described

**Cytotoxicity measurements in adult HDFa cells and epidermal keratinocytes**

To identify the effects of a mixture of fucoidan and olive leaf extract (oleuropein) on the proliferation of adult HDFa cells (human dermal fibroblasts) and epidermal keratinocytes (human epidermal keratinocyte HaCaT cells), an MTT assembly was used for an analysis. Adult HDFa cells and epidermal keratinocytes were each seeded into 96-well plates at a density of $1\text{-}3\times10^3$ cells/well and cultured for 24 hours, after which a stock solution 1000-fold concentrated of a reference mixture composed of fucoidan and olive leaf extract (oleuropein) at a 1:1 weight ratio as individually diluted with sterile distilled water to prepare working solutions at 50 fold, 25 fold, 10 fold, 1 fold, and 0.1 fold concentrations, and the working solutions were then added to the respective wells and incubated for an additional 24 hours. After incubation, an MTT solution was added and reacted, and the absorbance was measured at 540 nm.

As a result, when the cell viability (proliferation rate) of adult HDFa cells in the negative control group without introduction of the mixture was set to 100%, the cell viability (proliferation rate) after 24 hours of treatment with the mixture at each concentration was 98.38% at a 0.1 fold concentration, 98.10% at a 1 fold concentration, 88.40% at a 10-fold concentration, and 84.17% at a 25 fold concentration, showing no significant cytotoxicity, and human epidermal keratinocytes exhibited cell viability of 98.96% at a 0.1 fold concentration, 97.65% at a 1 fold concentration, 99.42% at a 10 fold concentration, 93.79% at a 25 fold concentration, and 86.36% at a 50 fold concentration of the mixture, also confirming no significant cytotoxicity (see FIG. 1).

**2. Measurement of wound healing efficacy in adult HDFa cells and epidermal keratinocytes**

To identify the effects of the mixture of fucoidan and olive leaf extract (oleuropein) on wound healing in adult HDFa cells and epidermal keratinocytes, an analysis was performed by using a wound healing assembly. Cells were seeded into 12-well plates at a density of $0.5\text{-}3\times10^5$ cells/well and cultured for 24 hours, after which a horizontal scratch was created by using a yellow tip, followed by two washes with medium to remove cells. A stock solution, which was a 1,000 fold concentrated preparation of a reference mixture composed of fucoidan and olive leaf extract (oleuropein) at a 1:1 weight ratio, was individually diluted with sterile distilled water to prepare working solutions at 50 fold, 25 fold, 10 fold, 1 fold, and 0.1 fold concentrations, which were then introduced into the respective wells and incubated for 24 hours. Following incubation, images of the surface area of the horizontal scratch were captured by a microscope at 0 and 24 hours, and the surface area of the scratch was measured by using the ImageJ program.

As a result, it was identified that treatment with the mixture of fucoidan and olive leaf extract (oleuropein) adjusted the scratch area in adult HDFa cells after 24 hours to 18.8% (wound healing rate of 81.2%) at a 0.1 fold concentration, 25.0% (wound healing rate of 75%) at a 1 fold concentration, and 34.3% (wound healing rate of 65.7%) at a 10 fold concentration, compared to 76.6% (wound healing rate of 23.4%) in the negative control group. It is recognized that the wound healing efficacy of the mixture was maximized at lower concentrations (see FIG. 2).

It is recognized that the scratch area of human epidermal keratinocytes after 24 hours was measured at 5.6% (wound healing rate of 94.4%) at a 0.1 fold concentration, 8.6% (wound healing rate of 91.4%) at a 1 fold concentration, and 11.7% (wound healing rate of 88.3%) at a 10 fold concentration of the mixture, compared to 39.8% (wound healing rate of 60.2%) in the negative control group, indicating that the wound healing efficacy of the mixture was also enhanced at lower concentrations (see FIG. 3]).

Accordingly, in the present disclosure, fucoidan is added in a small amount within the range of 0.2 to 3.0 wt% and

olive leaf extract is added in a small amount within the range of 0.2 to 3.0 wt% with respect to the total wound dressing composition.

Hereinafter, the contents of toxicity tests in an accredited testing institution on an embodiment composition arbitrarily selected within the ranges of glycerin 60 to 70 wt%, honey 28 to 39 wt%, fucoidan 0.2 to 3.0 wt%, olive leaf extract 0.2 to 3.0 wt%, and xanthan gum 0.1 to 0.3 wt% (at the midpoint concentrations of 65 wt% of glycerin, 33 wt% of honey, 0.9 wt% of fucoidan, 0.9 wt% of olive leaf extract, and 0.2 wt% of xanthan gum, as shown in Table 1 below.

[Table 1]

| | | | | |
|---|---|---|---|---|
| 1 | Skin sensitization test using guinea pigs | ’22.09.27. ~’22.11.29. | ISO 10993-10: 2021(E), Test for skin sensitization 6.5 Guinea pig Maximization test | Korea Testing Laboratory |
| 2 | Single-dose acute systemic toxicity test using mice | ’22.10.18. ~’22.11.29. | ISO 10993-11: 2017, Test for systemic toxicity | Korea Testing Laboratory |
| 3 | Cytotoxicity test using L929 cells | ’23.01.16. ~23.01.27. | ISO 10993: 2009, Biological Evaluation of Medical Devices - Part 5: Test for in vitro cytotoxicity ISO 10993: 2021, Biological Evaluation of Medical Devices - Part 12: Sample Preparation | Korea Testing Certification Institute |
| 4 | Intracutaneous reactivity test using rabbits (direct contact) | ’23.06.13. ~’23.08.01. | ISO 10993-23: 2021(E), 7.3 Animal irritation test by intracutaneous(intradermal) administration | Korea Testing Laboratory |
| 5 | Pyrogen test using rabbits. | ’23.06.13. ~’23.08.01. | ISO 10993-11:2017(E), Annex G. Information on material media ted pyrogens | Korea Testing Laboratory |
| 6 | Skin irritation test using rabbits (direct contact) | ’23.06.13. ~’23.08.01. | ISO 10993-23: 2021 (E), 7.2 Animal irritation test by skin exposure | Korea Testing Laboratory |
| 7 | Endotoxin test using lysate reagent (Colorimetric method) | ’23.06.21. ~23.07.19. | USP 40, <85> Bacterial Endotoxin s Test USP 43, <161> Medical Devices-Bacterial Endotoxin and Pyrogen Tests | Korea Testing Laboratory |

The contents of each test showed no toxicity associated with the composition of the embodiment the present disclosure, and the results for respective tests are as follows.

## 1. Test 1: Skin sensitization test using guinea pigs

This test was conducted to evaluate whether skin sensitization occurs after administering a test substance (a composition of the embodiment of the present disclosure) to experimental animals (female guinea pigs).

Primary intradermal induction, secondary topical induction, and tertiary challenge tests were conducted on groups of 5 or 10 animals each for the negative control substance and the test substance, during which mortality, symptoms, and body weight were observed and measured throughout the test period. Furthermore, skin reactions at all application sites were evaluated at 24 $\pm$ 2 hours and 48 $\pm$ 2 hours after removal of the tertiary patch.

As a result of the test, no experimental animals administered with either the negative control substance extract or the test substance extract died during the test period, and no symptoms, abnormal behaviors, or changes in body weight attributable to the administered substances were observed. Neither erythema nor edema was observed at the induction sites of all animals, and the grade on the Magnusson & Kligman scale was 0. In conclusion, the test substance was evaluated as non-sensitizing to the skin.

[Table 2]

| Negative control (NC) | |
|---|---|
| Type | 1) Sodium Chloride solution, 0.9%/ Saline: SC |

(continued)

| Negative control (NC) | |
|---|---|
| | 2) Cotton Seed Oil: CSO |
| Basis for selection | - ISO 10993-12: 2021(E). Sample preperation and reference materials |
| | - ISO 10993-10:2021 (E), Test for skin sensitization 6.5 Guinea pig Maximization test |
| Remarks | The extraction solvent for the test substance is the same as the above negative control substance |

[Table 3]

| Preparation of test substance | | | |
|---|---|---|---|
| Step | Extraction solvent | Extraction ratio | Extraction conditions |
| Intracutaneous induction | SC (Colorless, Clear, No Visible particulates) | 0.2 g/ml | 70±2 °C<br>24±2h |
| | CSO(Yellow. Clear. No Visible particulates) | | |
| Local induction | SC (Colorless, Clear, No Visible particulates) | | |
| | CSO(Yellow. Clear. No Visible particulates) | | |
| Induction | SC (Colorless, Clear, No Visible particulates) | | |
| | CSO(Yellow. Clear. No Visible particulates) | | |

[Table 4]

| Test method (5 animals in control group, 10 animals in treatment group) | |
|---|---|
| Primary intracutaneous induction | The administered substances (negative control substance and test substance) were introduced into sterile syringes and injected 0.1 ml each into three sites (A, B, C from head to tail direction) on both scapular areas of the shaved guinea pigs in the test group. |
| Secondary local induction | Approximately 0.7 ml of the administered substance was dropped onto gauze attached to a non-irritant film and applied to the primary administration site of each test group, then fixed with adhesive tape, and the adhesive tape and gauze were removed after 48 hours. |
| Tertiary induction | Approximately 0.5 ml of the administered substance was dropped onto gauze attached to a non-irritant film and applied to the left flank of all tertiary challenge test groups, then fixed with adhesive tape, and the adhesive tape and gauze were removed after 24 hours. |

**2. Test 2: Single-dose acute systemic toxicity test using mice**

The test substance extract and negative control substance used in this test were the same as those used in Test 1 above.

This test was conducted to evaluate the potential for toxic reactions following a single intraperitoneal administration of the test substance to experimental animals (male mice, specific pathogen free (SPF)), with the negative control substance and test substance extract each administered once to five mice per experimental group. During the test period, mortality, clinical symptoms, and body weight were observed and measured, and a gross necropsy was performed on the day of autopsy.

As a result of the test, none of the experimental animals administered with the negative control substance or the test substance died during the test period. No abnormal symptoms or behaviors attributable to the administered substances were observed in any group, and no changes in body weight considered to be caused by the administered substances were observed. No gross abnormalities considered to be caused by the administered substances were observed during the visual necropsy.

**3. Test 3: Cytotoxicity test using L929 cells.**

The present test was conducted to determine whether the test substance exhibits cytotoxicity by using L929 cells.

L929 cells were inoculated into a 6-well plate and incubated for 24 ± 2 hours in an incubator maintained at 37 ± 1

°C with 5 ± 1% $CO_2$. A culture medium containing agar, prepared by adding approximately 50 mL of fetal bovine serum (FBS) and 10 mL of penicillin-streptomycin to 440 mL of a minimum essential medium (MEM), was dispensed and solidified, followed by staining of the cells using neutral red.

Negative control substances were prepared by cutting three pieces of RM-C (high-density polyethylene film), selected according to ISO 10993, into 1.0 $cm^2$ sections and sterilizing them at 121 ± 2 °C for 20 minutes, and positive control substances were prepared by cutting three pieces of RM-B (0.25% ZDBC polyurethane film), also selected according to ISO 10993, into 1.0 $cm^2$ sections and sterilizing them at 121 ± 2 °C for 20 minutes. Test substances were prepared by sufficiently wetting three pieces of 0.45 μm filters measuring 1.0 $cm^2$ each with the composition of the embodiment of the present disclosure.

The negative control substances, positive control substances, and test substances prepared as described above were each applied to stained L929 cells, and upon observation, the negative control group exhibited no morphological changes in the cells beneath or around the samples, and no cell lysis was detected. Furthermore, because no areas of decolorization were observed, the cytotoxicity reaction was classified as grade 0. The positive control group exhibited cell rounding, with cell lysis and areas of decolorization extending up to 0.4 cm around the sample, resulting in a cytotoxicity reaction classified as grade 3. The experimental group exhibited cellular abnormalities and areas of decolorization confined directly beneath the sample, resulting in a cytotoxicity reaction classified as grade 2.

Based on the results identified in the negative and positive control groups, the test procedure was deemed appropriate, and considering the overall test results, the cytotoxicity reaction grade of the test substance was determined to be grade 2, indicating that the test substance is non-cytotoxic.

[Table 5]

| Cytotoxicity Test Using L929 Cells (Agar Diffusion Test) | | | |
|---|---|---|---|
| Classification | Reaction site (cm) (reactivity zone) | Reactivity | Grade |
| Test substance 1 | 0 | Mild | 2 |
| Test substance 2 | 0 | Mild | 2 |
| Test substance 3 | 0 | Mild | 2 |
| Negative control group 1 | 0 | None | 0 |
| Negative control group 2 | 0 | None | 0 |
| Negative control group 3 | 0 | None | 0 |
| Positive control group 1 | 0.4 | Moderate | 3 |
| Positive control group 2 | 0.4 | Moderate | 3 |
| Positive control group 3 | 0.4 | Moderate | 3 |

**4. Test 4: Intradermal reaction test using rabbits (direct contact).**

The test substance extract and negative control substance used in this test were the same as those used in Test 1 above.

This test was conducted to evaluate the potential irritancy induced by the test substance when a single administration of the test substance extract is performed intradermally (within the dermis) in female New Zealand White (NZW), SPF rabbits.

Extracts of the test substance eluted with physiological saline (SC) and cottonseed oil (CSO) were administered to three rabbits. On the shaved dorsal area of the rabbits centered on the spine, 0.2 mL of test substance extract prepared with each elution solvent (SC and CSO) was intradermally administered at five sites per solvent, totaling ten sites on the left side, and 0.2 mL of negative control substance prepared with each solvent (SC and CSO) was administered at five sites per substance, totaling ten sites on the right side.

During the test period, the mortality, symptoms, and body weight of the experimental animals were observed and measured.

As a result of the test, no animals died during the test period, and none of the animals exhibited any specific symptoms deemed to be caused by the administered substances. No changes in body weight considered to be caused by the administered substances were observed.

Intradermal reactions at the administration sites of the test substance extract and the negative control substance were observed, and intradermal reaction scores at each administration site were calculated at 24 ± 2 hours, 48 ± 2 hours, and 72 ± 2 hours after administration according to the observation criteria. A difference in average scores between

administration sites for each extract was 1.0 or less, and when the extracts were the same, no difference was observed between the test substance and the negative control substance.

No specific irritation was observed at the test substance administration sites, and thus the test substance is considered not to induce localized reactions in the intradermal tissues of rabbits.

[Table 6]

| Intracutaneous reaction score calculation criteria | |
|---|---|
| Reaction | Score |
| Erythema and scab Formation | |
| No Erythema | 0 |
| Very slight erythema (almost Imperceptible) | 1 |
| Distinct erythema (well-defined) | 2 |
| Moderate erythema | 3 |
| Severe erythema (deep red) ~ scab formation that is difficult to grade | 4 |
| Edema formation | |
| No edema | 0 |
| Very slight edema (almost imperceptible) | 1 |
| Well-formed edema (clearly swollen with distinct boundaries of the edematous area) | 2 |
| Moderate edema (approximately 1 mm swelling) | 3 |
| Severe edema (swelling over 1 mm and extending beyond the exposed area) | 4 |
| Maximum possible irritation score | 8 |
| Other abnormal changes appearing on the skin area will be recorded and reported. | |

[Table 7]

| Intracutaneous reaction score | | | | | | | | | | | | |
|---|---|---|---|---|---|---|---|---|---|---|---|---|
| Ext rac t sol utio n | Observation score | | | | | | | | | | | |
| | 24±2 hours after administration (day 1) | | | | 48±2 hours after administration (day 2) | | | | 72±2 hours after administration (day 3) | | | |
| | Test group | | Control group | | Test group | | Control group | | Test group | | Control group | |
| | Eryt hem a | Ed em a | Eryt hem a | Ed em a | Eryt hem a | Ed em a | Eryt hem a | Ed em a | Eryt hem a | Ed em a | Eryt hem a | Ed em a |
| SC | 0 | 0 | 0 | 0 | 0 | 0 | 0 | 0 | 0 | 0 | 0 | 0 |
| CS O | 1 | 1 | 1 | 1 | 1 | 1 | 1 | 1 | 1 | 1 | 1 | 1 |
| SC | 0 | 0 | 0 | 0 | 0 | 0 | 0 | 0 | 0 | 0 | 0 | 0 |
| CS O | 1 | 1 | 1 | 1 | 1 | 1 | 1 | 1 | 1 | 1 | 1 | 1 |
| SC | 0 | 0 | 0 | 0 | 0 | 0 | 0 | 0 | 0 | 0 | 0 | 0 |
| CS O | 1 | 1 | 1 | 1 | 1 | 1 | 1 | 1 | 1 | 1 | 1 | 1 |
| * Each score is the average of observation scores for each administration site (observation scores are the same at each administration site) | | | | | | | | | | | | |

**5. Test 5: Pyrogen test using rabbits.**

This test was conducted to verify the biological safety of the test substance by evaluating the potential pyrogenic response following a single administration of the test substance extract into the ear vein of female New Zealand White (NZW), SPF rabbits. The elution solvent used was SC.

The baseline body temperature was measured prior to administration, and the test substance extract was administered as a single intravenous injection into the ear vein of three rabbits at a dose of 10 mL/kg. Experimental animals were assigned to groups such that the difference in baseline body temperature between individual rabbits was less than 1.0 °C, and any animals with a baseline body temperature exceeding 39.8 °C were excluded from the test.

One hour after administration of the test substance, while the body temperature was measured five times at 30 minute intervals to measure the difference from the baseline body temperature, the mortality and symptoms were observed throughout the test period.

As a result of the test, none of the experimental animals administered with the test substance extract exhibited a rise in body temperature of 0.5°C or more compared to the control, indicating that the test substance is nonpyrogenic (among the three rabbits tested, two showed no increase in body temperature at all, and one exhibited a maximum increase of 0.2°C). In addition, during the test period, none of the experimental animals died, and no animals exhibiting abnormal symptoms were observed.

**6. Test 6: Skin irritation test using rabbits (Direct contact)**

This test is intended to evaluate the potential of the test substance to cause irritation when it is brought into direct contact with the skin of rabbits (strain: New Zealand White (NZW), SPF, female).

The test substance and the negative control substance were applied to the dorsal area of the shaved rabbits. In accordance with ISO 10993-23, the test substance was directly applied to the skin by dividing a 5.0 × 5.0 cm piece of sterile gauze into four equal parts to obtain four 2.5 × 2.5 cm sterile gauze pads, stacking them into twelve layers, and then dropping 0.5 ml of the test substance onto the gauze by using a syringe. The negative control substance was also applied in accordance with ISO 10993-23 by dividing a 5.0 × 5.0 cm piece of sterile gauze into four equal parts to obtain four 2.5 × 2.5 cm sterile gauze pads, stacking them into twelve layers, and then dropping 0.5 ml of sterile saline (SC) onto the gauze by using a syringe.

The application sites were wrapped with appropriate pressure that would not cause irritation to the abdomen and fixed with adhesive tape to prevent the applied test substance and the negative control substance from being displaced or lost. The test substance and the negative control substance were removed from the application sites 4 to 24 hours after application.

During the test period, the mortality, clinical symptoms, and body weight were observed and measured. In accordance with the observation criteria, skin reactions at the application sites were observed, and skin irritation scores were calculated at 1 ± 0.1 hours, 24 ± 2 hours, 48 ± 2 hours, and 72 ± 2 hours after removal of the applied substances.

As a result of the test, no animals died during the test period, and all animals showed no abnormal symptoms or changes in body weight that were deemed to be caused by the administered substances.

The primary irritation index (PII) of the test substance and negative control substance application sites was 0.0 (Negligible). In conclusion, the test substance is determined not to cause skin irritation in rabbits.

[Table 8]

| Primary irritation index (PII) | |
|---|---|
| Average score | Reaction category |
| 0.0-0.4 | Negligible |
| 0.5-1.9 | Slight |
| 2.0-4.9 | Moderate |
| 5.0-8.0 | Severe |

**7. Test 7: Endotoxin test using lysate reagent (Colorimetric method)**

This test is intended to quantitatively identify the endotoxin level of the test substance by using a lysate reagent.

The extraction solvent for the test substance was selected as endotoxin free reagent water (LRW) (LAL reagent water) in accordance with USP 40 and USP 43, and extraction was performed under the conditions shown in Table 9 below in accordance with ISO 10993-12.

[Table 9]

| Extraction solvent | Sample form | Extraction ratio | Amount of extraction solvent | Extraction conditions |
|---|---|---|---|---|
| LRW | Amorphous | 10 ml/ea | 40ml | 37±1 °C, 60±2 min |

The test substance extract was applied to a PTS cartridge and measured by using a PTS Reader. The channel information of the PTS cartridge is as follows.

[Table 10]

| Channel No. | Configuration of Channel |
|---|---|
| 1,3 | 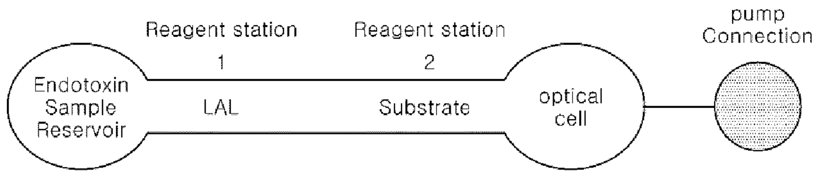  Sample Channel Zoom |
| 2,4 | 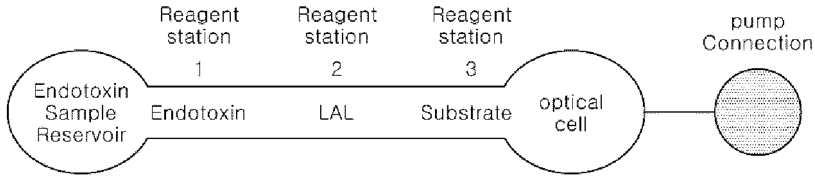  PPC (Spike) Channel Zoom |

The endotoxin specification value of the extract is calculated as follows by using the endotoxin specification value of the test substance (20 EU/device).

$$\text{Endotoxin release limit (ERL)}$$

$$= K \times N / V = 20\ EU \times 1 / 40\ ml = 0.5\ EU/ml$$

K : the Endotoxin limit per device (20 EU)
N : the number of devices tested
V : the total volume of water used to extract the endotoxin from the devices tested

By using the endotoxin specification value of the extract calculated as described above, the maximum valid dilution factor is obtained as follows.

Maximum valid dilution factor = ERL × concentration of test substance / $\lambda$ (most sensitive sensitivity of PTS cartridge) = 0.5 EU/ml × 1 / 0.005 EU/ml = 100 fold

The most sensitive sensitivity of the PTS cartridge is 0.005 EU/ml.
The concentration of the test substance is 1 (100% extract).
The experimental group is as follows.

[Table 11]

| Test group | Endotoxin concentration | Added test solution | Number of tests performed |
|---|---|---|---|
| A | 0 | Test solution | 2 |
| B | Concentration at the midpoint of the calibration curve | Test solution | 2 |

(continued)

| Test group | Endotoxin concentration | Added test solution | Number of tests performed |
|---|---|---|---|
| C | Concentrations of 3 or more | LRW | 2 per concentration |
| D | 0 | LRW | 2 |
| - A: Contains test solution only<br>- B: Test solution diluted to the same multiple as A, with endotoxin standard solution added to achieve the endotoxin concentration at or near the midpoint of the calibration curve<br>- C: Endotoxin standard solutions at concentrations of 3 or more used for the calibration curve reliability test<br>- D: Negative control containing LRW only | | | |

Group A was applied to channels 1 and 3 of the PTS cartridge, and Group B was applied to channels 2 and 4 of the PTS cartridge to measure the endotoxin recovery rate and identify the presence of reaction interference factors. The Crns and Group D were used to identify the calibration curve and the reliability of LRW by identifying the information included in the PTS cartridge.

After preparing the test substance extract as the test solution, the pH was measured by using pH-indicator paper (MERCK), resulting in a pH of 6.0 to 7.0 for the test solution. Twenty-five μl of the test solution were applied to each of the four channels of the PTS cartridge. Measurement was performed by using the PTS Reader at 37 ± 1 °C with an absorbance of 395 nm.

As a result of the test, the reaction time coefficient of variation (CV) for both the sample and spike was less than 25%, verifying the suitability of the test, the endotoxin recovery rate was within the range of 50 to 200%, determining no interfering substances in the test solution, and the endotoxin concentration of the test solution was below 0.5 EU/ml. Accordingly, under the present test conditions, the endotoxin concentration of the test substance extract used in the test was determined to be below 0.5 EU/ml.

[Table 12]

| Quantitative test results | |
|---|---|
| Classification | Result |
| Sample Rxn time CV | 4.3% |
| Spike Rxn Time CV | 1.5% |
| Spike Recovery | 52% |
| Test suitability | Pass |
| Sample value | 0.05 EU/ml |
| - Sample rxn time CV: The CV% of channels 1 and 3 (two repeats of A) indicates the difference between the two repeats and is considered acceptable when below 25%.<br>- Spike rxn time CV: The CV% of channel 2.4 (two repeats of B) indicates the difference between the two repeats and is considered acceptable when below 25%.<br>- Spike recovery (endotoxin recovery rate): When the endotoxin recovery rate is within the range of 50 to 200%, it is considered that no interfering factors are present.<br>- Test suitability: The validity is evaluated based on the CV% of A and B and the endotoxin recovery rate, and the test is considered valid when marked as "pass." When the endotoxin recovery rate is "fail," follow the instructions of the test supervisor.<br>Sample value: The endotoxin value represents the concentration reacted by the test solution, and when the test solution does not react completely, it is expressed as the reagent used multiplied by the dilution factor of λ. When the concentration is below the lowest range of the calibration curve, the sample value is displayed as below the most sensitive concentration of the PTS cartridge, and the test is considered valid when marked "pass" in the test suitability section. | |

Meanwhile, the viscosity of the wound dressing composition has be between 500 and 10,000 mPa·s (Ministry of Food and Drug Safety Notification No. 2023-2, Korean Pharmacopoeia General Test Methods, 57, Viscosity Measurement Method).

The viscosity of the composition of the present disclosure in the test example was measured to be 5,420 mPa·s, which is within the specified range.

[Table 13]

| Test conditions | Test results | |
|---|---|---|
| | Torque (%) | Viscosity (mPa-s) |
| - Test temperature: 23±0.2°<br>- Test method: Rotating drum titration method<br>- Spindle: SC4-21, 5 RPM<br>- Sampling time: 60 sec | 54.2 | 5,420 |

The following are the results of the wound induction test on experimental animals. This test is intended to comparatively evaluate the healing ability of the wound dressing composition of the present disclosure.

Experimental animals (mice, specific pathogen free (SPF)) were anesthetized with isoflurane inhalation anesthesia, the dorsal skin was disinfected, and wounds were induced by removing skin tissues by using a biopsy punch (Φ5 mm), after which the natural healing process was observed in the control group, and the healing progress was observed in the experimental group, to which the wound dressing composition (test substance) provided by the present disclosure was applied, and the control group and experimental group were compared. The test substance was applied evenly to the wound lesion and surrounding skin by impregnating sterile gauze (3 cm × 5 cm) (with 0.2 g per application), and an adhesive bandage (3 cm × 10 cm) was cut and wrapped around the abdomen and back of the experimental animal so that the gauze portion contacting the wound, thereby fixing the gauze in place covering both the abdomen and back of the experimental animal.

From day 1 to day 10 of the test, wound areas were measured once daily, and based on the measured values, the wound healing rate for each day and the healing ratio of the experimental group relative to the control group were calculated.

For wound area measurement, the gauze and bandage were cut and removed by using sterilized surgical scissors, the scab was removed with sterilized forceps to accurately measure the wound area (lesion site), bleeding was controlled by applying sterile gauze for one minute if present, and the wound area was calculated based on the major and minor axis lengths of the wound lesion measured by using a vernier caliper.

The composition of the wound dressing applied to the experimental group is as shown in Table 14 below, and it was applied once daily to each wound lesion and the surrounding skin in the manner described above.

[Table 14]

| Classification | Content of each component (wt%) |
|---|---|
| Glycerin | 65 |
| Honey | 33 |
| Fucoidan | 0.5 |
| Olive leaf extract | 1.3 |
| Xanthan gum | 0/2 |

FIG. 4 is a graph showing the wound healing rate by day for the control group and the experimental group, and FIG. 5 is a graph showing the daily healing ratio of the experimental group relative to the control group. FIG. 6 is a photograph depicting the wound healing status by date in a control group and an experimental group.

In FIG. 4, the experimental group exhibited a faster wound healing rate than the control group up to day 5, after which the healing rates of the experimental group and the control group were observed to be nearly similar. In FIG. 5, the recovery ratio of the experimental group compared to the control group rapidly increased until day 4, after which the difference in recovery rates gradually decreased. Accordingly, the experimental group is determined to contribute to accelerated wound healing.

Although the present disclosure has been described in connection with the above-mentioned test examples, various modifications and variations are possible within the scope that does not depart from the gist of the present disclosure, and it may be applied in various fields. Accordingly, the claims of the present disclosure include modifications and variations that fall within the true scope of the prior invention.

## Industrial Applicability

The present disclosure may be used as a wound dressing for the healing of wounds and burns and for the improvement of scars on human or animal skin.

## Claims

**1.** A wound dressing composition comprising:

60 to 70 wt% of glycerin;
28 to 39 wt% of honey;
0.2 to 3.0 wt% of fucoidan;
0.2 to 3.0 wt% of olive leaf extract; and
0.1 to 0.3 wt% of xanthan gum.

**2.** The wound dressing composition of claim 1, wherein the fucoidan is obtained by mixing dried seaweed stem powder or extract with purified water, followed by centrifugation, filtration, and collection.

**3.** The wound dressing composition of claim 1, wherein the olive leaf extract is obtained by subjecting a filtrate of crushed olive leaf powder or an extract from olive leaves to sterilization by heating at 120 to 125°C for 15 to 18 minutes.

**4.** The wound dressing composition of any one of claim 1 to claim 3, wherein a viscosity is adjusted to a range of 500 to 10,000 mPa·s depending on a content of xanthan gum.

FIG. 1

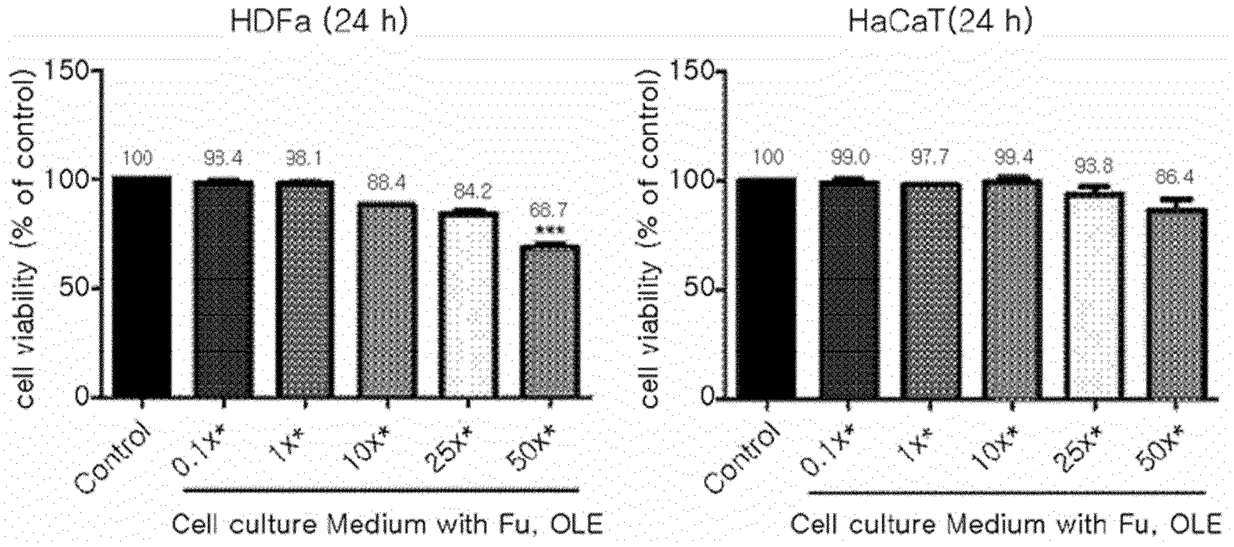
HDFa (24 h)
cell viability (% of control)
150
100
50
0
100
99.4
98.1
88.4
84.2
68.7
***
Control
0.1x*
1x*
10x*
25x*
50x*
Cell culture Medium with Fu, OLE
HaCaT(24 h)
cell viability (% of control)
150
100
50
0
100
99.0
97.7
99.4
93.8
86.4
Control
0.1x*
1x*
10x*
25x*
50x*
Cell culture Medium with Fu, OLE

FIG. 2

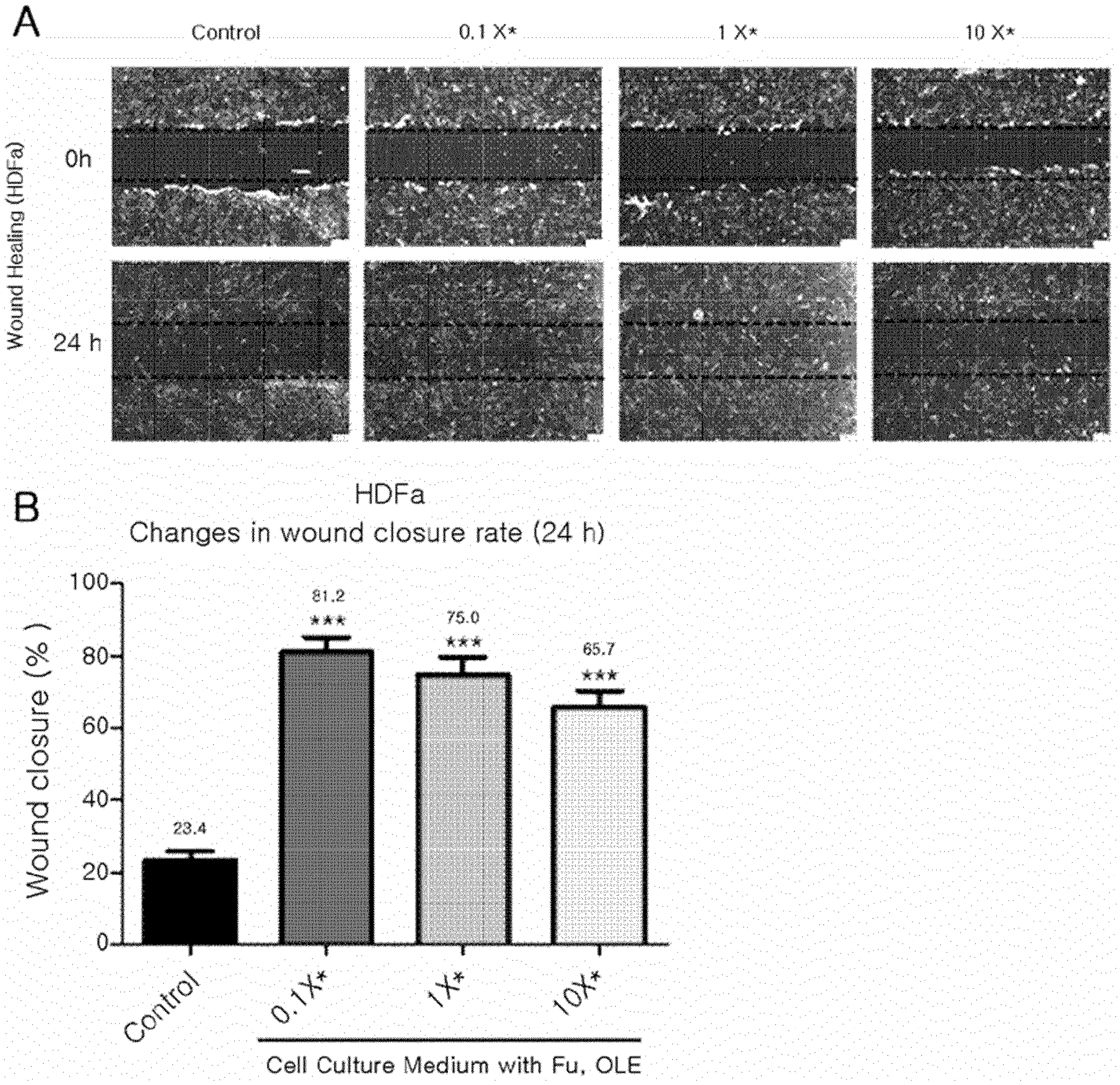
A
Control
0.1 X*
1 X*
10 X*
Wound Healing (HDFa)
0h
24 h
B
HDFa
Changes in wound closure rate (24 h)
Wound closure (%)
100
80
60
40
20
0
23.4
81.2
***
75.0
***
65.7
***
Control
0.1X*
1X*
10X*
Cell Culture Medium with Fu, OLE

FIG. 3

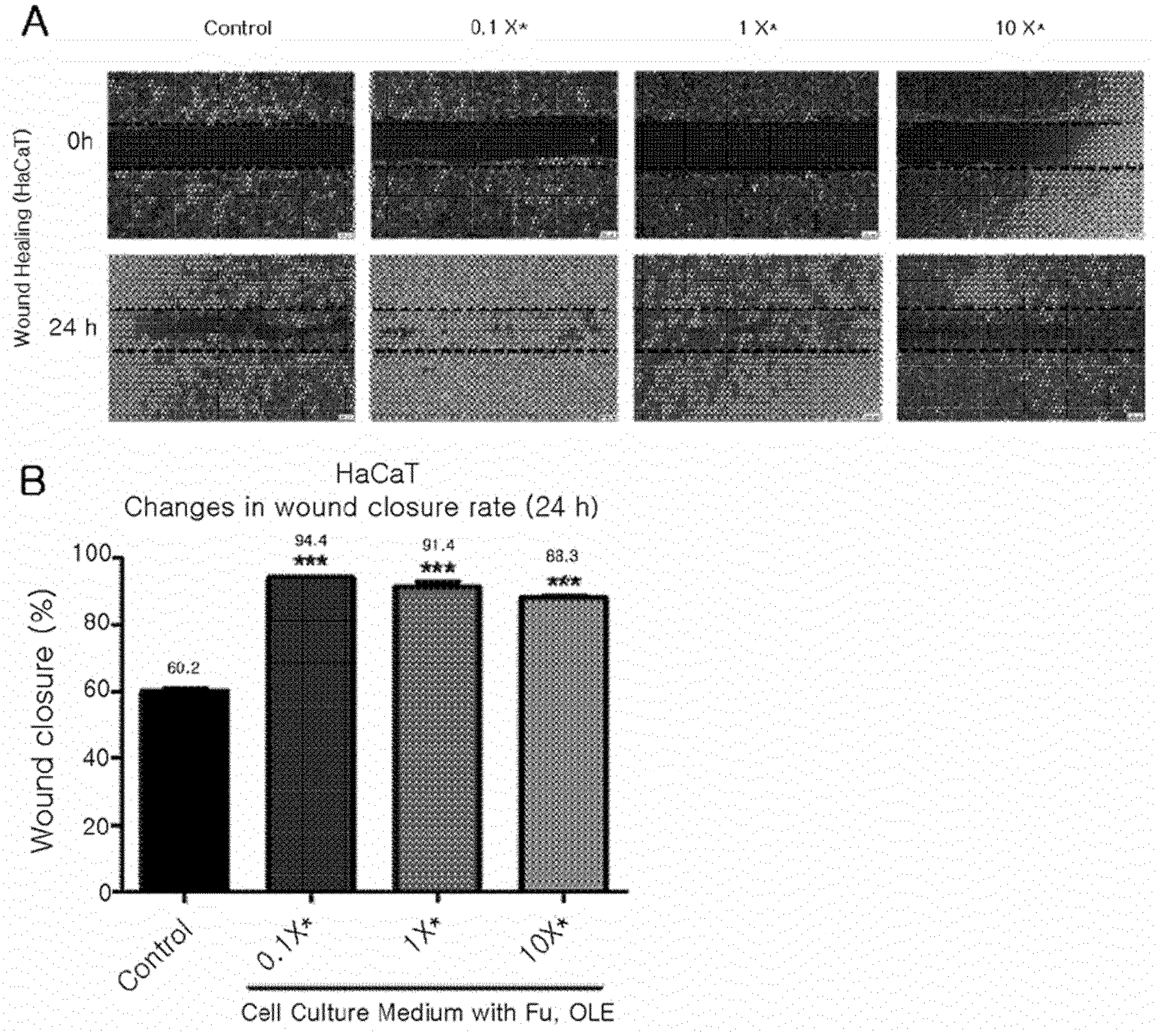
A
Control
0.1 X*
1 X*
10 X*
Wound Healing (HaCaT)
0h
24 h
B
HaCaT
Changes in wound closure rate (24 h)
Wound closure (%)
100
80
60
40
20
0
60.2
94.4
***
91.4
***
88.3
***
Control
0.1X*
1X*
10X*
Cell Culture Medium with Fu, OLE

FIG. 4

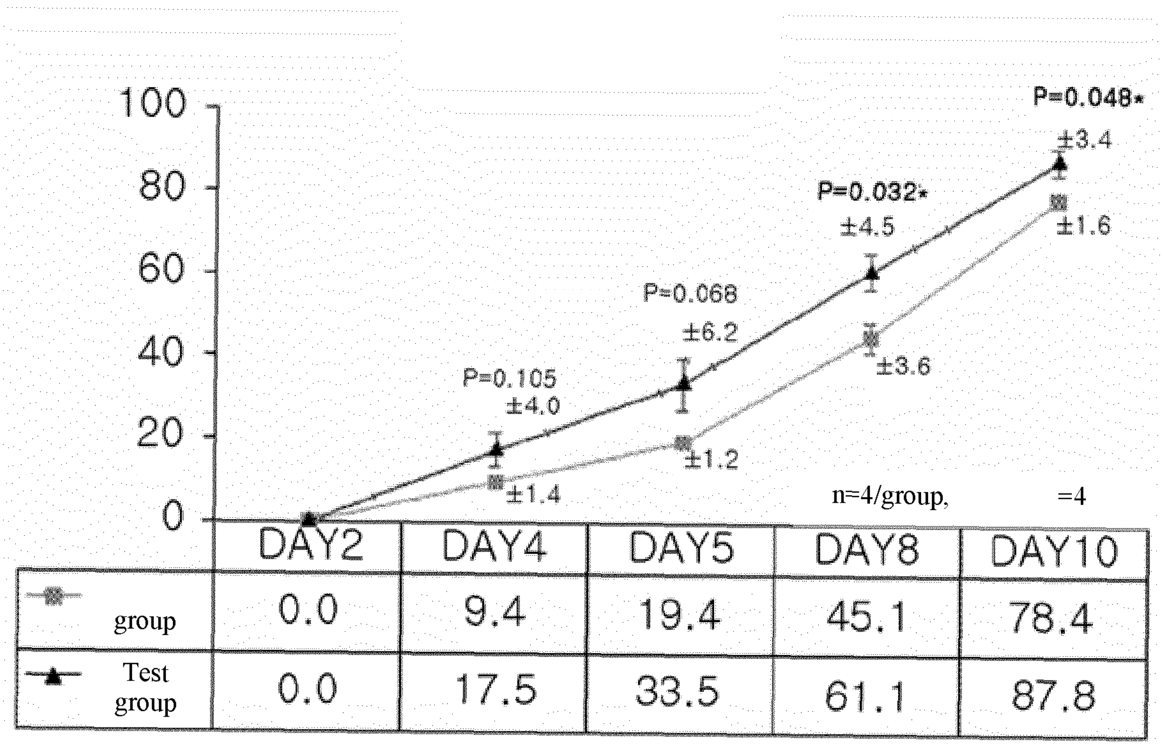


| | DAY2 | DAY4 | DAY5 | DAY8 | DAY10 |
|---|---|---|---|---|---|
| group | 0.0 | 9.4 | 19.4 | 45.1 | 78.4 |
| Test group | 0.0 | 17.5 | 33.5 | 61.1 | 87.8 |

FIG. 5

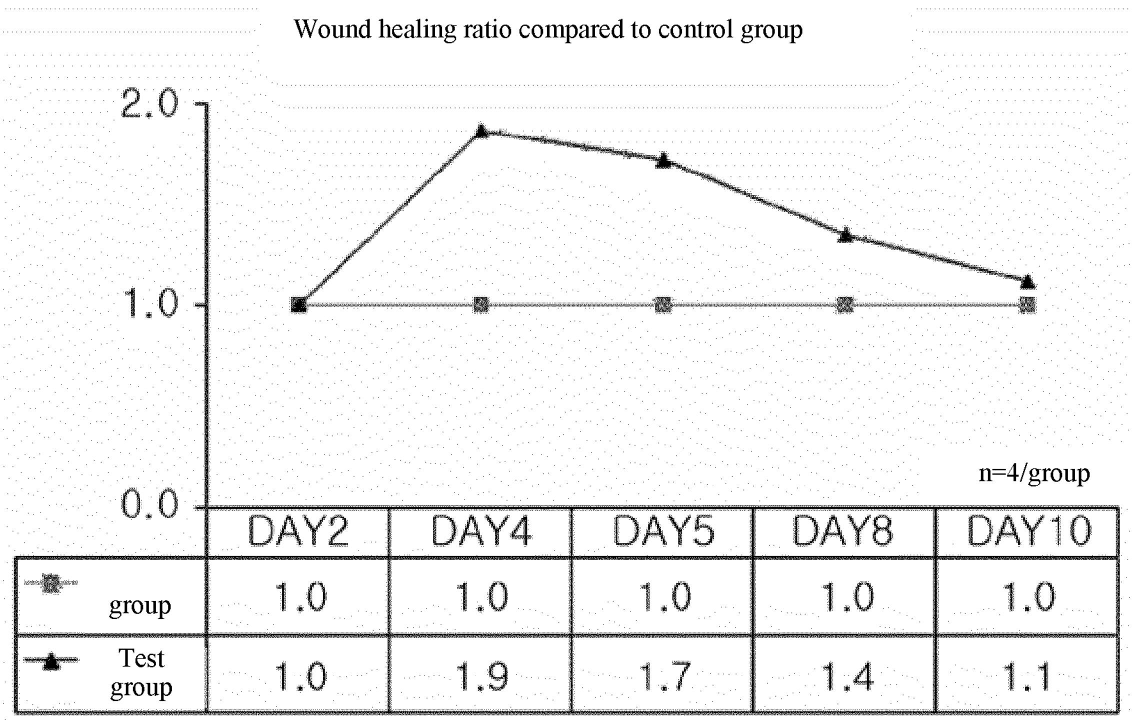


| | DAY2 | DAY4 | DAY5 | DAY8 | DAY10 |
|---|---|---|---|---|---|
| group | 1.0 | 1.0 | 1.0 | 1.0 | 1.0 |
| Test group | 1.0 | 1.9 | 1.7 | 1.4 | 1.1 |

FIG. 6

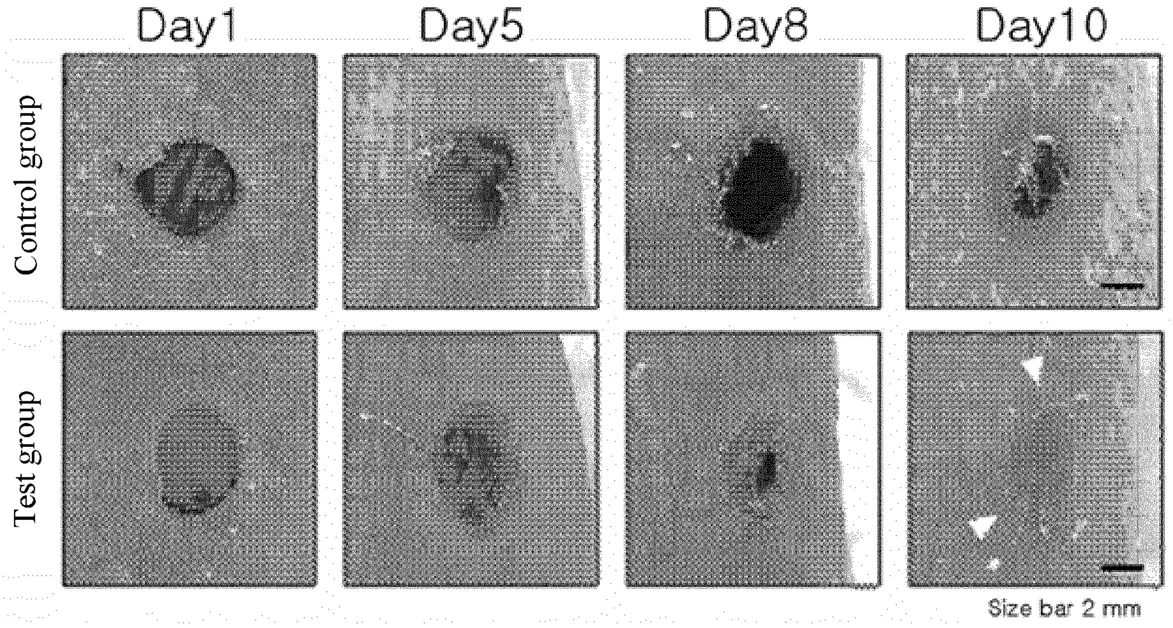

**INTERNATIONAL SEARCH REPORT**

International application No.
**PCT/KR2023/019323**

**A. CLASSIFICATION OF SUBJECT MATTER**

**A61L 26/00**(2006.01)i

According to International Patent Classification (IPC) or to both national classification and IPC

**B. FIELDS SEARCHED**

Minimum documentation searched (classification system followed by classification symbols)

A61L 26/00(2006.01); A61K 31/05(2006.01); A61K 31/365(2006.01); A61K 31/366(2006.01); A61K 36/28(2006.01); A61K 38/08(2006.01); A61K 8/97(2006.01); A61Q 19/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 창상 피복재(wound dressing), 글리세린(glycerin), 벌꿀(honey), 후코이단(fucoidan), 올리브나무잎추출물(olive tree leaf extract), 잔탄검(xanthan gum)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2014-0048258 A (STEMTECH INTERNATIONAL, INC.) 23 April 2014 (2014-04-23)<br>See paragraphs [0034]-[0040]; and table 3. | 1-4 |
| Y | KR 10-2022-0053266 A (LEE, Sang Chul) 29 April 2022 (2022-04-29)<br>See paragraphs [0069]-[0075] and [0078]. | 1-4 |
| A | ERDOGAN, Ipek et al. Wound healing effects of various fractions of olive leaf extract (OLE) on mouse fibroblasts. Romanian Biotechnological Letters. 2018, vol. 23, no. 6, pp. 14217-14228.<br>See entire document. | 1-4 |
| A | KR 10-2009-0028836 A (DSM IP ASSETS B.V.) 19 March 2009 (2009-03-19)<br>See entire document. | 1-4 |
| A | US 2017-0224760 A1 (ALASTIN SKINCARE, INC.) 10 August 2017 (2017-08-10)<br>See entire document. | 1-4 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 March 2024** | **27 March 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**
**Information on patent family members**

International application No.
**PCT/KR2023/019323**

| Patent document cited in search report | Publication date (day/month/year) | Patent family member(s) | Publication date (day/month/year) |
|---|---|---|---|
| KR 10-2014-0048258 A | 23 April 2014 | EP 2739155 A1 | 11 June 2014 |
| | | EP 2739155 A4 | 07 October 2015 |
| | | JP 2014-521698 A | 28 August 2014 |
| | | US 2014-0227363 A1 | 14 August 2014 |
| | | US 9289375 B2 | 22 March 2016 |
| | | WO 2013-022788 A1 | 14 February 2013 |
| KR 10-2022-0053266 A | 29 April 2022 | KR 10-2446051 B1 | 21 September 2022 |
| | | WO 2022-086047 A1 | 28 April 2022 |
| KR 10-2009-0028836 A | 19 March 2009 | CN 101516364 A | 26 August 2009 |
| | | CN 101516364 B | 28 December 2011 |
| | | EP 2040696 A2 | 01 April 2009 |
| | | EP 2040696 B1 | 25 January 2017 |
| | | JP 2010-500964 A | 14 January 2010 |
| | | JP 5339373 B2 | 13 November 2013 |
| | | KR 10-1435228 B1 | 28 August 2014 |
| | | US 2010-0056463 A1 | 04 March 2010 |
| | | US 8841264 B2 | 23 September 2014 |
| | | WO 2008-006581 A2 | 17 January 2008 |
| | | WO 2008-006581 A3 | 05 June 2008 |
| US 2017-0224760 A1 | 10 August 2017 | CN 108883048 A | 23 November 2018 |
| | | EP 3411012 A1 | 12 December 2018 |
| | | EP 3411012 A4 | 28 August 2019 |
| | | JP 2019-504083 A | 14 February 2019 |
| | | JP 2022-017351 A | 25 January 2022 |
| | | JP 6966455 B2 | 17 November 2021 |
| | | JP 7150120 B2 | 07 October 2022 |
| | | KR 10-2018-0114911 A | 19 October 2018 |
| | | US 10086035 B2 | 02 October 2018 |
| | | US 10286030 B2 | 14 May 2019 |
| | | US 10688147 B2 | 23 June 2020 |
| | | US 11426442 B2 | 30 August 2022 |
| | | US 11426443 B2 | 30 August 2022 |
| | | US 2018-0289767 A1 | 11 October 2018 |
| | | US 2019-0262418 A1 | 29 August 2019 |
| | | US 2020-0338154 A1 | 29 October 2020 |
| | | US 2021-0205405 A1 | 08 July 2021 |
| | | WO 2017-136600 A1 | 10 August 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 102088767